Europäisches Patentamt

European Patent Office

Office européen des brevets

(11) Veröffentlichungsnummer: **0 205 179 B1**

## EUROPÄISCHE PATENTSCHRIFT

(12)

(19)

(45) Veröffentlichungstag der Patentschrift: **26.08.92**

(21) Anmeldenummer: **86108055.4**

(22) Anmeldetag: **12.06.86**

(51) Int. Cl.⁵: **C12M 3/00**, C12N 13/00,
//C12N15/00

---

(54) Kammer für die Behandlung von Zellen im elektrischen Feld.

---

(30) Priorität: **12.06.85 DE 3521034**
**25.06.85 DE 3522610**

(43) Veröffentlichungstag der Anmeldung:
**17.12.86 Patentblatt 86/51**

(45) Bekanntmachung des Hinweises auf die
Patenterteilung:
**26.08.92 Patentblatt 92/35**

(84) Benannte Vertragsstaaten:
**FR GB NL**

(56) Entgegenhaltungen:
**EP-A- 0 126 389**
**EP-A- 0 225 636**
**DE-U- 8 517 148**

(73) Patentinhaber: **Forschungszentrum Jülich
GmbH
Postfach 1913
W-5170 Jülich(DE)**

(72) Erfinder: **Matschke, Christian
Wirthstrasse 63
W-5110 Alsdorf(DE)**

## Beschreibung

Die Erfindung bezieht sich auf eine Kammer für die Behandlung von Zellen im elektrischen Feld mit einem zur Aufnahme der die Zellen enthaltenden Suspension vorgesehenen Raum mit einer zwischen zwei Elektroden liegenden Zone, innerhalb der die Zellen einem zwischen den Elektroden ausgebildeten elektrischen Feld ausgesetzt werden.

Eine Kammer der eingangs bezeichneten Art ist aus der DE-OS 33 17 415 bekannt. Bei dieser bekannten Kammer wird der zur Aufnahme der Zellsuspension vorgesehene Raum durch einen zylinderförmigen Innenkörper und einen diesen umgebenden Außenkörper gebildet. Die beiden Elektroden umgeben den Innenkörper in Form einer mehrgängigen Schraube, so daß die Zone, innerhalb der die Zellen dem elektrischen Feld ausgesetzt werden, als den Innenkörper in Form einer mehrgängigen Schraube umgebenden Teilbereich ausgebildet ist. Diese Ausführungsform der Kammer bietet zwar die Möglichkeit, eine sehr große Zahl von Zellen zugleich einer elektrischen Behandlung zu unterziehen. Ein gewisser Nachteil besteht dabei jedoch darin, daß konstruktionsbedingt nicht alle in der Zone befindlichen Zellen der gleichen elektrischen Feldstärke ausgesetzt werden. Dies erhöht die ohnehin durch die natürliche Streuung der Eigenschaften einer Zellpopulation bedingte Schwankungsbreite im Hinblick auf das gewünschte Produkt. Darüberhinaus ist diese bekannte Kammer aufwendig in der Herstellung und daher kostspielig.

Eine Kammer der eingangs bezeichneten Art ist ferner aus der DE-OS 33 21 239 bekannt. Diese besteht aus einer Grundplatte mit einem den Raum für die Zellsuspension bildenden weiteren Teil, wobei in den Raum zwei drahtförmige Elektroden hineinragen. Zwar ist diese Ausführungsform der Kammer leicht und preiswert herstellbar. Bei der zwischen den Elektroden liegenden Zone treten jedoch die gleichen Nachteile wie bei der vorgenannten Kammer auf.

Kammern der beschriebenen Art sind generell einsetzbar für die Behandlung von Zellen im elektrischen Feld, insbesondere auch zur Fusion von Zellen.

Ein Verfahren zur Fusion von Zellen ist aus Biochimica et Biophysica Acta, 694 (1982), 227 - 277 (Electric Field-Mediated Fusion and Related Electrical Phenomena, U. Zimmermann) bekannt. Bei diesem bekannten Verfahren - dessen Ablauf unter dem Mikroskop beobachtet werden kann - wird der Membrankontakt zwischen wenigstens zwei Zellen durch Anlegen eines alternierenden, schwach homogenen Feldes erzeugt. Durch das elektrische Feld werden, bedingt durch Polarisationsprozesse in der Zelle, Dipole erzeugt, die sich gegenseitig anziehen, wenn sich die Zellen während ihrer Wanderung im elektrischen Feld einander nähern (sog. Dielektrophorese). Nach der Bildung der Zellenreihe werden die Störungen in der Membranstruktur zwischen benachbarten Zellen durch einen elektrischen Durchbruchpuls ausgelöst (J. Membrane Biol. 67, 165 - 182 (1982), Electric Field-Induced Cell-to-Cell Fusion, U. Zimmermann and J. Vienken). Dabei werden - nach den bisherigen Modellvorstellungen - Löcher in der Membrankontaktzone benachbarter Zellen erzeugt, die zu einem zytoplastischen Kontinuum zwischen den beiden Zellen und zur Brückenbildung von Lipiden zwischen den Membranen der benachbarten Zellen führen. Die Lipidomoleküle ordnen sich nicht mehr in ihre ursprüngliche Membran ein. Sobald sich eine Brücke gebildet hat, kommt es aus energetischen Gründen zur Abrundung des entstandenen Gebildes, das aus den über die Lipidbrücken miteinander verbundenen Zellen besteht.

Es ist Aufgabe der Erfindung, eine Kammer zu schaffen, die einfach herzustellen und leicht handhabbar ist und bei der der oben erwähnte Nachteil der bekannten Kammern vermieden ist.

Diese Aufgabe wird erfindungsgemäß durch eine Kammer der eingangs bezeichneten Art gelöst, bei der die Zone - bis auf eine Einfüll- bzw. Ausfüllöffnung für die Zellsuspension - von äquidistant einander gegenüberliegenden, koaxial oder konzentrisch angeordneten Oberflächen der Elektroden begrenzt ist. Die Zone ist damit durch die Elektroden räumlich abgegrenzt. In jedem Bereich der Zone ist, da sie ja durch äquidistante Oberflächen der Elektroden umfaßt wird, die elektrische Feldstärke gleich, so daß für alle in der Zone befindlichen Zellen die gleichen elektrischen Bedingungen gegeben sind. Im Bereich der Zone ist damit zugleich auch der zur Aufnahme der Zellsuspension vorgesehene Raum mit der Zone identisch.

Bei einer ersten besonderen Ausführungsart der Kammer gemäß der Erfindung ist im Bereich der Zone die erste Elektrode als Innenelektrode mit zylindrischer oder im wesentlichen zylindrischer Oberfläche und die zweite Elektrode als Außenelektrode mit nach innen gerichteter zylindrischer oder im wesentlichen zylindrischer Oberfläche ausgebildet, so daß die Zone ein zylindrischer oder nahezu zylindrischer Ringraum ist. Die geometrische Form des Ringraumes kann dabei insofern von der zylindrischen Form abweichen, als der Querschnitt nicht exakt kreisförmig sein muß, sondern beispielsweise auch oval sein kann.

Eine zweckmäßige Ausgestaltung der ersten Ausführungsart der Kammer besteht darin, daß die eine Stirnseite des Ringraumes nach außen offen ist und daß sich an der anderen Stirnseite des Ringraumes zumindest eine radial in der Innenelektrode verlaufende Querbohrung anschließt, die in einen axialen Kanal mündet, der in der Innenelektrode verläuft und der zu der der Ausfüllöffnung entgegengesetzten Seite der Kammer nach außen offen ist.

Zur Vorbereitung der elektrischen Behandlung der Zellen wird die Zellsuspension über den Kanal in die Zone eingedrückt, in der sie - bei üblichen Elektrodenabständen von 20 bis 500 µm - verbleibt, ohne herauszu tropfen. Nach der elektrischen Behandlung kann die Zellsuspension durch Nachdrücken von Gas (Luft) oder einer zweiten Lösung oder durch Zentrifugieren aus der Zone herausgedrückt werden.

Um beide Elektroden in einfacher Weise an die elektrische Versorgung anschließen zu können, sind - bei der ersten Ausführungsart der Kammer - die beiden Elektroden außerhalb des Bereichs des Ringraumes durch ein Isolierteil voneinander getrennt, das bis zur Querbohrung reicht, einen Teil der Innenelektrode im Bereich des axialen Kanals umfaßt und die beiden Elektroden bis zur Außenseite der Kammer voneinander trennt, so daß die Außenelektrode im wesentlichen Bereich des Ringraumes und die Innenelektrode im übrigen Teil dar Kammer von außen zugänglich sind. Die elektrischen Anschlüsse können bei dieser Ausführungsform auf einfache Weise von außen an die Kammer angelegt werden, wobei es für die Durchführung der Fusion von Zellen in der Regel um Anschlüsse einer elektrischen Einrichtung zur Erzeugung eines alternierenden elektrischen Feldes und einer elektrischen Einrichtung zur Erzeugung elektrischer Spannungsimpulse handelt.

Eine weitere, sehr vorteilhafte Ausführungsform der ersten Ausführungsart der Kammer besteht darin, daß der axiale Kanal in einen sich nach außen hin erweiternden Innenkonus mündet, dessen Abmessungen so gewählt sind, daß in ihn eine Pipette eingeführt werden kann. Über die Pipette kann auf diese Weise eine an das Volumen der Zone angepaßte Menge an Zellsuspension in die Kammer eingeführt werden. Dies ermöglicht eine sehr einfache Handhabung der Kammer, wobei bei Vorhandensein einer Vielzahl von Kammern auf sehr einfache Weise eine Serie von Behandlungen von Zellen, beispielsweise ganze Untersuchungsreihen an einer oder mehrerer Zellsuspensionen durchgeführt werden können.

Um die Kammern in eine elektrische Steckverbindung, die den Kontakt mit der elektrischen Versorgung herbeiführt, leicht einführen zu können, ist es ferner zweckmäßig, daß die Außenelektrode zur Seite der Ausfüllöffnung des Ringraumes hin sich verjüngend konisch ausgebildet ist.

Bei einer zweiten Ausführungsart der Kammer gemäß der Erfindung ist die erste Elektrode als Innenelektrode ausgebildet und im Bereich der Zone kugelförmig oder nahezu kugelförmig und die zweite Elektrode als Außenelektrode ausgebildet, wobei sie im Bereich der Zone eine der Oberfläche einer Kugel entsprechende oder nahezu entsprechende Innenoberfläche aufweist, so daß die Zone ein zwischen zwei konzentrischen Kugeloberflächen oder nahezu kugeligen Oberflächen liegender Raum ist.

Die als Innenoberfläche ausgebildete erste Elektrode ist zweckmäßigerweise stabförmig mit an einem Ende angebrachtem Kugelkopf ausgebildet. Diese Innenelektrode ragt in eine in der Form eines Reagenzglases ausgebildete Außenhülle hinein, wobei im Bereich der Zone die innere Oberfläche der Außenhülle zugleich die innere Oberfläche der Außenelektrode ist.

Zur Vorbereitung der elektrischen Behandlung der Zellen wird die Innenelektrode in die reagenzglasförmige Außenhülle eingebracht und erst dann die Zellsuspension oder eine Lösung und anschließend eine kleine Menge an Zellsuspension eingefüllt. Für den Fall, daß die eingefüllte Lösungsmenge größer ist, als es dem Volumen der Zone entspricht, sammeln sich die Zellen durch Sedimentation im Bereich der Zone an.

Zur Führung der Innenelektrode in der reagenzglasförmigen Außenhülle sind am stabförmigen Teil der Innenelektrode Führungsringe angebracht, die die Innenelektrode in dem zylindrischen Teil der reagenzglasförmigen Außenhülle führen.

Um den elektrischen Kontakt von außen an die Außenelektrode anlegen zu können, ist die Außenhülle im Bereich der Zone durch die Außenelektrode gebildet. Die Außenelektrode kann dabei vom übrigen Teil der Außenhülle durch ein Isolierteil getrennt sein. Alternativ kann der übrige Teil der Außenhülle auch aus einem elektrisch isolierenden Material bestehen. Der zweite elektrische Kontakt liegt am stabförmigen Teil der Innenelektrode an.

Eine zweckmäßige Ausführungsform der zweiten Ausführungsart der Kammer gemäß der Erfindung besteht darin, daß an dem dem Kugelkopf entgegengesetzten Ende der Innenelektrode ein Verschluß für die in der Form eines Reagenzglases ausgebildete Außenhülle angebracht ist.

Für den Fall, daß die Außenhülle im Bereich der Zone durch die Außenelektrode gebildet wird und der übrige Teil der Außenhülle aus einem elektrisch isolierenden Material besteht, besteht der Verschluß aus einem Metall, so daß der Anschluß der elektrischen Spannung an die Innenelektrode über diesen metallischen Verschluß erfolgen kann.

Zur leichteren Handhabung der Kammer ist am Verschluß eine Öffnung mit einem Stopfen vorgesehen. Durch diese Öffnung wird die Zellsuspension eingefüllt. Zur Entnahme der Zellsuspension nach erfolgter elektrischer Behandlung der Zellen wird der Verschluß abgenommen und damit die Innenelektrode aus der reagenzförmigen Außenhülle herausgenommen. Die weitere Behandlung der Zellsuspension erfolgt dann wie bei jedem Reagenzglas üblich.

In Erweiterung der Ausführungsform der ersten Ausführungsart der Kammer gemäß der Erfindung, bei der ein Anschluß für eine Pipette vorgesehen ist, ist die Kammer gemäß der Erfindung dadurch gekennzeichnet, daß die Zone eine nach außen offene Ausfüllöffnung aufweist und die Zone, zumindest aber deren Bereich um die Ausfüllöffnung so bemessen sind, daß die Zellsuspension durch Kapillarwirkung in der Zone gehalten wird und wobei die Zone eine Einfüllöffnung aufweist, die mit einem Anschluß für eine Pipette o.dgl. verbunden ist.

Der Bereich der Ausfüllöffnung kann beispielsweise auch in Form von kapillaren Kanälen gestaltet sein.

Bei dieser Ausführungsart der Kammer gemäß der Erfindung kann die Zone sehr unterschiedlich gestaltet sein. Lediglich die Einfüllöffnung mit dem für eine Pipette o.dgl. vorgesehenen Anschluß und die nach außen offene Ausfüllöffnung und die kapillare Bemessung des Bereichs dieser Ausfüllöffnung sind dann vorgegeben. So kann beispielsweise die Zone so gestaltet sein, wie sie aus der DE-OS 33 17 415 oder auch aus der DE-OS 33 21 239 bekannt ist.

Auch kann beispielsweise bei der zweiten Ausführungsart der Kammer gemäß der Erfindung, bei der eine reagenzglasförmige Außenhülle vorgesehen ist, diese Außenhülle nach unten kapillare Kanäle aufweisen und am Verschluß dieser Kammer bzw. an dessen Öffnung ein Zusatz für den Anschluß einer Pipette vorgesehen sein. Diese Version der Kammer wird dann mittels einer Pipette befüllt und durch Eindrücken einer zweiten Lösung (ebenfalls mittels der Pipette) über die Kanäle nach unten entleert.

Das Verfahren zum Befüllen und Entleeren einer entsprechend gestalteten Kammer besteht somit darin, daß zum Befüllen der Kammer bzw. der Zone Zellsuspension über den Anschluß mittels einer Pipette o.dgl. gedrückt wird und daß zum Entleeren der Kammer bzw. der Zone die behandelte Zellsuspension über die Kammer bzw. Zone aus dieser hinausgedrückt wird. Dieses Verfahren ermöglicht eine besonders einfache Handhabung der entsprechenden Fusionskammer.

Die beiden ersten Ausführungsarten der Kammer gemäß der Erfindung sind in der Zeichnung schematisch dargestellt und werden im folgenden näher erläutert:

Es zeigen

Figur 1 die erste Ausführungsart, welche mittels einer Pipette befüllbar ist,

Figur 2 die zweite Ausführungsart der Kammer mit reagenzglasförmiger Außenhülle und stabförmiger Innenelektrode mit Kugelkopf.

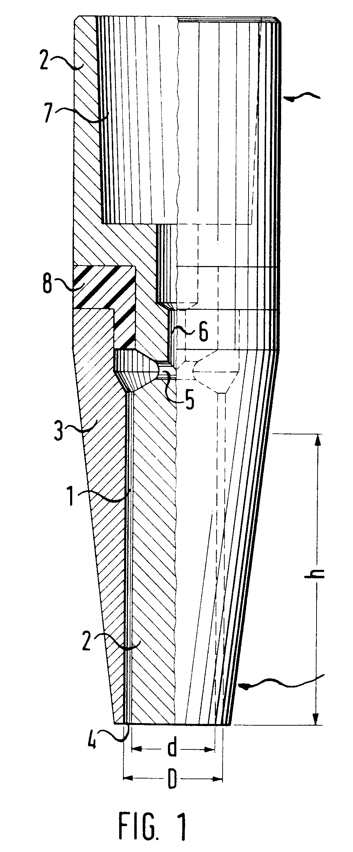

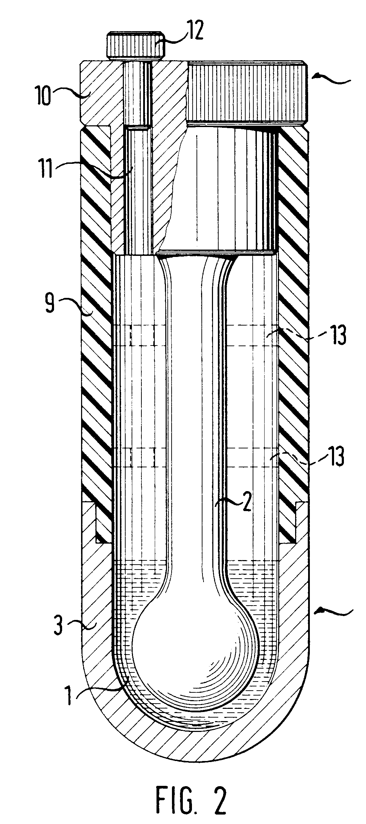

Beide Ausführungsarten der Kammer weisen, wie aus den Figuren 1 und 2 ersichtlich ist, eine Zone 1 auf, die - bis auf eine Einfüll- bzw. Ausfüllöffnung - je nach Ausführungsart durch koaxial oder konzentrisch angeordnete Oberflächen der Elektroden 2 und 3 begrenzt ist.

Bei der in Figur 1 dargestellten Ausführungsart der Kammer ist die Innenelektrode 2 im Bereich der Zone 1 als zylindrischer Körper ausgebildet und von der Außenelektrode 3 derart umgeben, daß die Zone 1 ein zylindrischer Ringraum ist. Dieser Ringraum ist an einer Stirnseite nach außen (unten) offen und hat hier seine Ausfüllöffnung 4. An der anderen Stirnseite schließt sich an den Ringraum 1 eine radial, in der Innenelektrode 2 verlaufende Querbohrung 5 an, die in einen axialen, in der Innenelektrode 1 angeordneten Kanal 6 mündet. An diesen Kanal schließt sich ein sich nach außen hin erweiternder Konus 7 an, dessen Abmessungen so gewählt sind, daß eine Pipette eingeführt werden kann.

Innenelektrode 2 und Außenelektrode 3 sind durch ein Isolierteil 8 voneinander getrennt. Die Außenelektrode 3 ist im Bereich der Zone 1 und die Innenelektrode 2 im oberen Bereich der Kammer von außen zugänglich. Die Außenelektrode ist außerdem konisch, nach unten sich verjüngend, ausgebildet. Die Kammer kann dadurch an die zur Durchführung der elektrischen Behandlung der Zellen benötigten elektrischen Spannungen angelegt werden, indem sie in eine der Kammerform entsprechende Steckverbindung eingesetzt wird.

Mittels der Pipette kann eine genau dosierte Menge an Lösung, in der die Zellen suspendiert sind, in die Zone eingedrückt werden. Durch Einfüllen einer weiteren, ebenfalls genau dosierten Lösungsmenge wird die behandelte Zellsuspension nach unten durch die Ausfüllöffnung 4 aus der Kammer - beispielsweise in ein Reagenzglas - gedrückt.

Beispielsweise bei Abmessungen von D = 4,4 mm, d = 4,0 mm und einer Höhe der Zone von h = 12 ergibt sich ein Zonenvolumen von ca. 32 $\mu$l bei einem Elektrodenabstand von 0,2 mm.

Bei der in Figur 2 dargestellten Ausführungsart der Kammer ist die Innenelektrode 1 stabförmig mit einem Kugelkopf ausgebildet. Die Außenhülle der Kammer weist die Form eines Reagenzglases auf, sie besteht im unteren Teil aus der Außenelektrode 2 und ist im oberen zylindrischen Teil 9 aus Kunststoff gefertigt.

An den stabförmigen Teil der Innenelektrode 2 schließt sich der Verschluß 10 an, der aus Metall besteht. Der Verschluß weist eine Öffnung 11 mit Stopfen 12 auf. Zur Führung der Innenelektrode im zylindrischen Teil der reagenzglasförmigen Außenhülle sind Führungsringe 13 vorgesehen.

Zur Durchführung der elektrischen Behandlung der Zellen wird die elektrische Spannung von außen im Bereich der Zone 1 an die Außenelektrode und an die Innenelektrode über den Verschluß 10 angelegt.

Bei einem Durchmesser der Kugel von 19,6 mm und einem inneren Durchmesser der Außenelektrode von 20 mm ergibt sich bei einem Elektrodenabstand von 0,2 mm ein Zonenvolumen von etwa 250 µl.

Zur Vorbereitung der elektrischen Behandlung der Zellen bei einer Kammer der angegebenen Abmessungen werden über die Öffnung 11 etwa 250 µl Lösung und danach 10 bis 200 µl Zellsuspension eingefüllt. Nachdem die Zellen in die Zone durch Sedimentieren gelangt sind, wird die Kammer für die Zeit der Durchführung der elektrischen Behandlung an die elektrische Versorgung angeschlossen. Anschließend wird die Innenelektrode aus der Außenhülle herausgenommen und die hierin befindliche Lösung in üblicher Weise weiterbehandelt.

**Patentansprüche**

1. Vorrichtung für die Behandlung von Zellen in elektrischen Feld mit zwei Elektroden, die im Bereich einer Zone des für die die Zellen enthaltende Suspension von außen zugänglichen Raumes gleichbleibende Abstände zueinander aufweisen,
   **dadurch gekennzeichnet,**
   daß die beiden Elektroden im Bereich der Zone (1) als Innen- bzw. Außenelektrode ausgebildet sind, wobei die Außenelektrode (3) die Innenelektrode (2) derart umschließt, daß die einander im gleichen Abstand gegenüberliegenden Flächen der Elektroden die Wände der Zone (1) bilden und daß die Innenelektrode durch ein sich an die Außenelektrode anschließendes, beide Elektroden voneinander trennendes Isolierteil (8, 9) geführt ist, über das hinaus sie sich in ein von außen zugängliches Teil erstreckt.

2. Vorrichtung nach Anspruch 1,
   **dadurch gekennzeichnet,**
   daß die Innenelektrode (2) im Bereich der Zone (1) mit zylindrischer oder im wesentlichen zylindrischer Oberfläche und die Außenelektrode (3) mit nach innen gerichteter zylindrischer oder im wesentlichen zylindrischer Oberfläche ausgebildet sind, so daß die Zone (1) ein zylindrischer oder nahezu zylindrischer Ringraum ist.

3. Vorrichtung nach Anspruch 2,
   **dadurch gekennzeichnet,**
   daß die eine Stirnseite des Ringraumes (1) nach außen offen ist (4) und daß sich an der anderen Stirnseite des Ringraumes zumindest eine radiale, in der Innenelektrode (2) verlaufende Querbohrung (5) anschließt, die in einen axialen Kanal (6) mündet, der im Bereich des Isolierteils (8) in der Innenelektrode (2) verläuft und der im von außen zugänglichen Teil der Innenelektrode in eine Einfüllöffnung mündet.

4. Vorrichtung nach Anspruch 3,
   **dadurch gekennzeichnet,**
   daß der axiale Kanal (6) in eine sich nach außen hin erweiternden Innenkonus (7) mündet, dessen Abmessungen so gewählt sind, daß in ihn eine Pipette eingeführt werden kann.

5. Vorrichtung nach Anspruch 4,
   **dadurch gekennzeichnet,**
   daß die Außenelektrode (3) zur Seite der Ausfüllöffnung (4) des Ringraumes (1) hin sich verjüngend konisch ausgebildet ist.

6. Vorrichtung nach Anspruch 1,
   **dadurch gekennzeichnet,**
   daß die Innenelektrode (2) im Bereich der Zone (1) kugelförmig oder nahezu kugelförmig ist und daß die Außenelektrode (3) im Bereich der Zone (1) eine der Oberfläche einer Kugel entsprechende oder nahezu entsprechende Innenoberfläche aufweist, so daß die Zone ein zwischen zwei konzentrischen Kugeloberflächen oder nahezu kugeligen Oberflächen liegender Raum ist.

7. Vorrichtung nach Anspruch 6,
   **dadurch gekennzeichnet,**
   daß die Innenelektrode (2) stabförmig mit an einem Ende angebrachtem Kugelkopf ausgebildet ist.

8. Vorrichtung nach Anspruch 6 oder 7,
   **dadurch gekennzeichnet,**
   daß die Innenelektrode (2) in eine in der Form eines Reagenzglases ausgebildete Außenhülle (3 und 9) hineinragt, wobei im Bereich der Zone (1) die Innenoberfläche der Außenhülle zugleich die Innenoberfläche der Außenelektrode (3) ist.

9. Vorrichtung nach Anspruch 8,
   **dadurch gekennzeichnet,**
   daß am stabförmigen Teil der Innenelektrode (2) Führungsringe (13) angebracht sind.

10. Vorrichtung nach Anspruch 8 oder 9,

**dadurch gekennzeichnet,**
daß die Außenhülle (3 und 9) im Bereich der Zone (1) durch die Außenelektrode (3) gebildet wird.

11. Vorrichtung nach Anspruch 10,
**dadurch gekennzeichnet,**
daß die Außenelektrode (3) vom übrigen Teil der Außenhülle durch das Isolierteil getrennt ist.

12. Vorrichtung nach Anspruch 10,
**dadurch gekennzeichnet,**
daß der übrige Teil der Außenhülle das Isolierteil (9) ist.

13. Vorrichtung nach einem der Ansprüche 7 bis 10 sowie wahlweise nach Anspruch 11 oder 12,
**dadurch gekennzeichnet,**
daß an dem dem Kugelkopf entgegengesetzten Ende der Innenelektrode (2) das von außen zugängliche Teil der Innenelektrode als Verschluß (10) für die in der Form eines Reagenzglases ausgebildete Außenhülle (3 und 9) ausgebildet ist.

14. Vorrichtung nach Anspruch 13,
**dadurch gekennzeichnet,**
daß der Verschluß (10) eine Öffnung (11) mit Stopfen (12) aufweist.

15. Vorrichtung nach einem der Ansprüche 1 bis 6,
**dadurch gekennzeichnet,**
daß die Zone (1) eine nach außen offene Ausfüllöffnung (4) aufweist und die Zone, zumindest aber deren Bereich um die Ausfüllöffnung so bemessen ist, daß die Zellsuspension durch Kapillarwirkung in der Zone gehalten wird.

16. Vorrichtung nach Anspruch 15, **dadurch gekennzeichnet,** daß der Bereich der Ausfüllöffnung in der Form von kapillaren Kanälen gestaltet ist.

**Claims**

1. Device for the treatment of cells in an electrical field, with two electrodes which in the region of a zone of that space which is accessible from the outside for the cells-containing suspension are situated at constant spacings from one another, characterised in that the two electrodes are constructed as an inner and outer electrode respectively in the region of the zone (1), the outer electrode (3) so surrounding the inner electrode (2) that the electrode surfaces situated opposite one another at the same spacing form the walls of the zone (1), and that the inner electrode extends through an insulating part (8, 9) which connects with the outer electrode and separates the two electrodes from each other and beyond which it extends into a part accessible from the exterior.

2. Device according to claim 1, characterised in that the inner electrode (2) in the region of the zone (1) is constructed with a cylindrical or substantially cylindrical surface, and the outer electrode (3) with an inwardly directed cylindrical or substantially cylindrical surface, so that the zone (1) is a cylindrical or approximately cylindrical annular space.

3. Device according to claim 2, characterised in that one end of the annular space (1) is open (4) to the exterior, and that the other end of the annular space connects with at least one radial transverse bore (5) which is arranged in the inner electrode (2) and which opens into an axial duct (6), which duct is arranged in the region of the insulating part (8) in the inner electrode (2) and in the part of the inner electrode which is accessible from the outside opens into a filling aperture.

4. Device according to claim 3, characterised in that the axial duct (6) opens into an outwardly widening inner cone (7) whose dimensions are so chosen that a pipette can be inserted into it.

5. Device according to claim 4, characterised in that the outer electrode (3) is given a conical shape tapering towards the emptying aperture (4) of the annular space (1).

6. Device according to claim 1, characterised in that in the region of the zone (1) the inner electrode (2) is spherical or approximately spherical in shape, and that the outer electrode (3) has in the region of the zone (1) an inner surface which corresponds or approximately corresponds to the surface of a sphere, so that the zone is a space situated between two concentric spherical or approximately spherical surfaces.

7. Device according to claim 6, characterised in that the inner electrode (2) is bar-shaped with a spherical head arranged on one end.

8. Device according to claim 6 or 7, characterised in that the inner electrode (2) projects into an outer envelope (3 and 9) which is made in the form of a test tube, and in the region of the zone (1) the inner surface of the outer envelope is at the same time the inner surface of the outer electrode (3).

9. Device according to claim 8, characterised in that guide rings (13) are arranged on the bar-shaped part of the inner electrode (2).

10. Device according to claim 8 or 9, characterised in that the outer envelope (3 and 9) is formed of the outer electrode (3) in the region of the zone (1).

11. Device according to claim 10, characterised in that the outer electrode (3) is separated from the remaining part of the outer envelope by the insulating part.

12. Device according to claim 10, characterised in that the remaining part of the outer envelope is the insulating part (9).

13. Device according to one of claims 7 to 10, also selectively according to claim 11 or 12, characterised in that at that end of the inner electrode (2) which is opposite from the spherical head that part of the inner electrode which is accessible from the exterior is constructed as a closure (10) for the outer envelope (3 and 9),the latter being made in the form of a test tube.

14. Device according to claim 13, characterised in that the closure (10) has an aperture (11) provided with a stopper (12).

15. Device according to one of claims 1 to 6, characterised in that the zone (1) has an outwardly open emptying aperture (4), and the zone, but at least the region thereof situated around the emptying aperture, is so dimensioned that the cell suspension is held in the zone by capillary action.

16. Device according to claim 15, characterised in that the region of the emptying aperture is made in the form of capillary ducts.

**Revendications**

1. Dispositif pour traiter des cellules dans un champ électrique, comportant deux électrodes qui sont séparées par des distances constantes au niveau d'une zone de l'espace accessible de l'extérieur pour la suspension de cellules,
   caractérisé par le fait que les deux électrodes sont agencées, au niveau de la zone (1), sous la forme d'une électrode intérieure et d'une électrode extérieure, l'électrode extérieure (3) entourant l'électrode intérieure (2) de telle sorte que les surfaces, situées en vis-à-vis à une même distance, des électrodes forment les parois de la zone (1) et que l'électrode intérieure traverse une partie isolante (8,9), qui se raccorde à l'électrode extérieure et sépare l'une de l'autre les deux électrodes et au-delà de laquelle l'électrode intérieure s'étend par une partie accessible de l'extérieur.

2. Dispositif selon la revendication 1, caractérisé en ce que l'électrode intérieure (2) est réalisée, au niveau de la zone (1), avec une surface cylindrique ou sensiblement cylindrique, et l'électrode extérieure (3) est réalisée avec une surface cylindrique ou sensiblement cylindrique dirigée vers l'intérieur, de sorte que la zone (1) est un espace annulaire cylindrique ou presque cylindrique.

3. Dispositif selon la revendication 2, caractérisé en ce qu'une face frontale de l'espace annulaire (1) s'ouvre à l'extérieur (4) et qu'à l'autre face frontale de l'espace annulaire se raccorde au moins un perçage transversal radial (5), qui s'étend dans l'électrode intérieure (2) et débouche dans un canal axial (6) qui, au niveau de la partie isolante (8), s'étend dans l'électrode intérieure (2) et qui débouche, dans la partie accessible de l'extérieur de l'électrode intérieure, dans une ouverture de remplissage.

4. Dispositif selon la revendication 3, caractérisé en ce que le canal axial (6) débouche dans un cône intérieur (7) qui s'élargit vers l'extérieur et dont les dimensions sont choisies de manière qu'on puisse y insérer une pipette.

5. Dispositif selon la revendication 4, caractérisé en ce que l'électrode extérieure (3) est agencée avec une forme conique se rétrécissant en direction du côté de l'ouverture de sortie (4) de l'espace annulaire (1).

6. Dispositif selon la revendication 1, caractérisé en ce que l'électrode intérieure (2) possède une forme sphérique ou approximativement sphérique au niveau de la zone (1) et que l'électrode extérieure (3) possède, au niveau de la zone (1), une surface intérieure qui correspond ou correspond approximativement à la surface d'une sphère de sorte que la zone est un espace situé entre deux surfaces sphériques ou presque sphériques, concentriques.

7. Dispositif selon la revendication 6, caractérisé en ce que l'électrode intérieure (2) est agencée en forme de tige, à une extrémité duquel est installée une tête sphérique.

8. Dispositif selon la revendication 6 ou 7, caractérisé en ce que l'électrode intérieure (2) pénètre dans une enveloppe extérieure (3 et 9) réalisée sous la forme d'un tube à essais, auquel cas, au niveau de la zone (1), la surface intérieure de l'enveloppe extérieure constitue simultanément la surface intérieure de l'électrode extérieure (3).

9. Dispositif selon la revendication 8, caractérisé en ce que des anneaux de guidage (13) sont montés sur la partie en forme de tige de l'électrode intérieure (2).

10. Dispositif selon la revendication 8 ou 9, caractérisé en ce que l'enveloppe extérieure (3 et 9) est formée, au niveau de la zone (1), par l'électrode extérieur (3).

11. Dispositif selon la revendication 10, caractérisé en ce que l'électrode extérieure (3) est séparée de la partie restante de l'enveloppe extérieure, par la partie isolante.

12. Dispositif selon la revendication 10, caractérisé en ce que la partie restante de l'enveloppe extérieure constitue la partie isolante (9).

13. Dispositif selon l'une des revendications 7 à 10 ainsi que, au choix, selon la revendication 11 ou 12, caractérisé en ce qu'à l'extrémité, située à l'opposé de la tête sphérique, de l'électrode intérieure (2), la partie accessible de l'extérieur de l'électrode intérieure est réalisée sous la forme d'un dispositif de fermeture (10) pour l'enveloppe extérieure (3 et 9) réalisée sous la forme d'un tube à essais.

14. Dispositif selon la revendication 13, caractérisé en ce que le dispositif de fermeture (10) possède une ouverture (11) munie d'un bouchon (12).

15. Dispositif selon l'une des revendications 1 à 6, caractérisé en ce que la zone (1) possède une ouverture de remplissage (4) ouverte vers l'extérieur, et que la zone, ou tout au moins sa partie entourant l'ouverture de sortie, doit être dimensionnée de telle sorte que la suspension de cellules est maintenue par effet capillaire dans la zone.

16. Dispositif selon la revendication 15, caractérisé en ce que la zone de l'ouverture de sortie est agencées sous la forme de canaux capillaires.

FIG. 1

FIG. 2